Europäisches Patentamt

(19) European Patent Office

Office européen des brevets

(11) Publication number: **0 260 315 B1**

(12) **EUROPEAN PATENT SPECIFICATION**

(45) Date of publication of patent specification:
11.12.91 Bulletin 91/50

(51) Int. Cl.⁵: **G01N 33/50, G01N 33/49,** // G01N33/52

(21) Application number: 87902238.2

(22) Date of filing: 04.03.87

(86) International application number:
PCT/US87/00523

(87) International publication number:
WO 87/05393 11.09.87 Gazette 87/20

(54) SEPARATION AND METHOD OF USE OF DENSITY SPECIFIC BLOOD CELLS.

(30) Priority: 10.03.86 US 838264

(43) Date of publication of application:
23.03.88 Bulletin 88/12

(45) Publication of the grant of the patent:
11.12.91 Bulletin 91/50

(84) Designated Contracting States:
AT BE CH DE FR GB IT LI LU NL SE

(56) References cited:
EP-A- 0 190 488
WO-A-86/03007
US-A- 4 190 535
Biological Abstracts, vol. 81, no.5, May 1986,(Philadelphia, PA.,US)J. Matsumoto et al.:"Clinical evaluation of fluorescein polarization of peripheral lymphocytes (SCM test) in the diagnosis of cancer", page AB-687, abstract 45158, J.Jpn.Soc.Cancer Ther.20(4):728-734, 1985
Biological Abstracts, vol.62, no.9, September 1976, (Philadelphia, PA.,US),L.Cercek et al.:"Changes in the structuredness of cytoplasmic matrix (SCM) induced in mixed lymphocyte interactions", page 4818, abstract 48873, Radiat. Environ.Biophys 13(1), 71-74, 1976

(56) References cited:
Biological Abstracts, vol.67, no.6,June 1977, (Philadelphia, PA., US)L.Cercek et al.:"Effects of osmolality and density of gradients on the isolation of SCM-responding lymphocytes" pages 3483 -3484, abstract 35241, Br.J.Cancer 38(1): 163-165,1978
Biological Abstracts, vol. 76, no.11, November 1983, (Philadelphia, PA., US) R.J.Atkinson et al.: "An analysis of the structuredness of cytoplasmic matrix test in cancer diagnosis", page 8978, abstract 81695, Cancer (Phila) 52(1), 91-100,1983

(73) Proprietor: CERCEK, Boris
4318 Camphor Avenue
Yorba Linda, CA 92686 (US)
Proprietor: CERCEK, Lea
4318 Camphor Avenue
Yorba Linda, CA 92686 (US)

(72) Inventor: CERCEK, Boris
4318 Camphor Avenue
Yorba Linda, CA 92686 (US)
Inventor: CERCEK, Lea
4318 Camphor Avenue
Yorba Linda, CA 92686 (US)

(74) Representative: Arthur, John William et al
FITZPATRICKS 4 West Regent Street
Glasgow G2 1RS Scotland (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Field of the Invention

This invention relates to the separation and method of use of density specific cells from human and animal blood.

Background of the Invention

Many diseases occurring in humans and animals can be detected by the presence of foreign substances, particularly in the blood, which are specifically associated with a disease or condition. Tests for antigens produced as a result of such diseases show great promise as a diagnostic tool for the early detection and treatment of the particular disease which produced the antigen. However, procedures for the detection of antigens must be reliable, reproducible and sensitive in order to constitute a practical diagnostic procedure for health care providers. In addition, any such procedure should be able to be carried out by persons of ordinary skill and training in laboratory procedures. For example, in the treatment of the various malignancies that afflict animal and human bodies, referred to generally as cancer, it is recognized that early detection is the key to effective treatment. In this connection new tests and procedures are being developed to effect early diagnosis of the cancer. We have developed and reported one such procedure for the early detection of cancer, L. Cercek, B. Cercek, C.I.V. Franklin, "Biophysical Differentiation Between Lymphocytes from Healthy Donors, patients with Malignant Diseases and Other Disorders," Brit. J. Cancer, Vol. 29, 345 (1974), L. Cercek, B. Cercek, "Application of the phenomenon of Changes in the Structuredness of Cytoplasmic Matrix (SCM) in the Diagnosis of Malignant Disorders: A review," Europ. J. Cancer, Vol. 13, 903-915 (1977). This procedure is based upon the response exhibited by a certain subpopulation of lymphocytes when they are exposed to mitogens and cancer associated antigens. Cell response is referred to herein as a change in the structuredness of the cytoplasmic matrix (SCM). Such changes in the cell SCM are measured by intercellular fluorescein fluorescence polarization in living cells. We found that lymphocytes from healthy donors and from donors with non-malignant diseases exhibit different SCM responses and can be distinguished on the basis of the SCM response from lymphocytes of donors afflicted with a cancer. The SCM response of the lymphocytes is induced by incubation of the cells with phytohaemaqglutinin (PHA), cancer basic proteins (CaBP) and(or antigens derived from specific malignant tumors (tumor associated antigen, TAA). As reported, the SCM test indicates usefulness both as a screening test and as a test to diagnose specific types of malignancies. This has been confirmed by blind clinical tests and corroborated by other investigators. We have found that the SCM test procedure is also useful for the detection of other antigen producing diseases such as viral infections, including the detection of the AIDS virus. The SCM test procedure may be used for monitoring other conditions that may not be classified as a disease, such as monitoring organ transplant rejection, tissue typing and allergenic reactions to various substances.

In order to successfully carry out the SCM test, however, it is imperative that the subpopulation of lymphocytes that exhibit the SCM response be isolated from other blood components, such as SCM non-responding lymphocytes. As we have reported, not all lymphocytes in blood are suitable for use in the SCM test procedure. Thus, in accordance with the procedure, SCM responders were isolated by centrifugation of the blood sample over a Ficoll®-Triosil density gradient solution having a specific density of 1.0810g/cm³ at 25°C and osmolality of 0.320 Osm/kg. The SCM responders separate as a narrow band on the specified density gradient and are collected with a Pasteur pipette, taking great care to avoid the collection of blood components which are separated above the narrow band of SCM responders or cells that separate in the density gradient solution below the narrow band. In addition, the conditions for isolation and separation of the SCM responders such as the temperature of the blood, the density of the density gradient solution and the osmolality and pH of the solution must be rigorously controlled and these conditions are critical to effecting the separation of the SCM responding sub-population of lymphocytes. In accordance with our procedure, temperature should be controlled ± 0.2°C and unless great care is exercised with temperature control, it is highly likely that some of all of the SCM responding cells will be missed which can result in a loss of sensitivity and in a worst case an indication of a false negative result. We have found that some others attempting to corroborate our work have failed to obtain reliable and reproducible results because of poor or unskilled technique in the separation of the SCM responding lymphocytes, a highly critical step in the SCM test procedure.

Summary of the Invention

In accordance with the present invention there is provided an improved procedure for the detection of disease and certain conditions in human and animal bodies based upon changes in the structuredness of the cytoplasmic matrix (SCM) of lymphocytes derived from the patient's blood in response to contact with disease or body condition associated substances such as antigens and mitogens where the lymphocytes utilized are SCM responding cells of a preselected narrow range of specific buoyant density. The invention further provides an improved method for separating the SCM responding lymphocytes from the donor's blood.

The lymphocytes used in the procedure of the invention are characterized by their SCM response to substances associated with a condition or disease and by having a buoyant density of $1.0590g/cm^3$ to $1.0670g/cm^3$ or a buoyant density of $1.0690g/cm^3$ to $1.0730g/cm^3$ at 20°C in a multi-density gradient solution having an osmolality of 0.315 Osm/kg.

The SCM responding cells are separated from blood in accordance with the invention by centrifugation with a multi-density gradient fluid having a minimum density at 20°C of not more than $1.050g/(cm^3$.which increases to at least $1.080g/cm^3$ and which has an osmolality of between 0.315 and 0.320 Osm(kg. In this manner the SCM responding cells are retained in the gradients corresponding to their buoyant densities and since the gradients increase in density, SCM responding lymphocytes in a preselected narrow density range are separated from the SCM non-responding cells and other blood components in a band which extends over a larger linear density interval, thus rendering the technique for separating the SCM responding cells more easily practiced by less skilled individuals and more readily adapted to automated techniques.

## Brief Description of the Drawings

Fig. 1 is a plot of density versus volume fraction of a continuous density gradient solution used in the invention; and

Fig. 2 is a plot of density versus volume fraction showing the volume fraction and density range of the gradient in which SCM responding cells are located.

## Description of the Specific Embodiments

The SCM test for the detection of cancer, reported in the European Journal of Cancer cited above, measures changes in the physical state of organization of the cytoplasmic matrix in living cells after incubation with PHA and CaBP or TAA. The term "structuredness of the cytoplasmic matrix," or SCM, is used to describe the physical state of organization of the cytoplasmic matrix of a cell at the molecular level. The SCM reflects the forces of interaction between macromolecules such as water molecules, ions, adenosine triphosphate, cyclic adenosine phosphate and other cardinal absorbants and perturbations of these interactions result in changes in the SCM. Such changes in SCM of lymphocytes have been found to be induced by mitogens such as PHA and by cancer associated antigens and cancer basic proteins (CaBP). Most importantly, we have found that animal and human SCM responding lymphocytes from donors afflicted with cancer exhibit differential SCM responses to PHA and CaBP derived from a pooled mixture of various tumor tissues from SCM responders of lymphocytes in healthy donors or donors afflicted with non-cancerous diseases. In addition, we have found that SCM responding lymphocytes from a donor afflicted with cancer respond only to antigens specifically derived from the same type of cancer as the donor's cancer and not to antigens specifically derived from a different type of cancer. These antigens are referred to as specific tumor associated antigens (TAA). Accordingly, the SCM test is useful as a diagnostic tool or as a screening test, depending upon the nature of the cancer associated antigen to which the lymphocytes are exposed.

Expanding on the results of the SCM test for cancer, we have found that SCM responding lymphocytes appear to, in a sense, be primed to exhibit an SCM response to any condition or disease associated substance with which they have come into contact by virtue of the, presence of that substance producing disease or condition in the host body from which the SCM responding lymphocytes were taken. Thus, the SCM test procedure can be applied to the detection of any antigen producing disease or condition. Moreover, in certain cases a patient may be afflicted with more than one condition or disease, for example, with both a cancer and a viral infection. The SCM test procedure and the SCM responding lymphocytes separated in accordance with the invention may be used to test both for the cancer and the viral infection simply by running two SCM tests using the SCM responders separated from the patient's blood but utilizing a cancer associated antigen in one test and an antigen associated with the virus in the other test. Similarly, the SCM test may be utilized to monitor the onset of rejection in organ transplant operations. By periodically running an SCM test on the patient's blood, the onset of organ rejection will be detected early enough to begin treatment and it will be unnecessary to administer medication to the patient before it is actually required. The term "antigen" is used herein in its broadest sense to designate any foreign disease or condition related substance which evokes the SCM response in SCM responding lymphocytes.

SCM responders are most conveniently measured by fluorescence polarization. fluorescein molecules are introduced into the living SCM responding cell and the degree of fluorescence polarization is measured. A high degree of polarization indicates a high SCM and vice versa. The change in polarization between control cells and those exposed to antigen indicates the SCM response of the cell. With respect to the SCM test for cancer, SCM responding lymphocytes from donors not afflicted with cancer exhibit a substantial SCM reduction when contacted by cancer associated antigens, CaBP or TAA. On the other hand, SCM responding lymphocytes from donors afflicted with cancer, regardless of the specific type of malignancy, exhibit substantially no response to PHA but do respond to CaBP and cancer associated antigens in general and specifically to the TAA derived from the same type of malignancy as that of the donor. These SCM responding cells comprise a first group of SCM responding lymphocytes referred to herein as F2 responders for reasons which will become apparent from the description of the method for separating the cells of this group.

As a result of the improved separation technique of the invention, we are able to separate a second group of SCM responding cells, referred to herein as F4 responders. This second group exhibits a different SCM response than the F2 responders in that when derived from donors free of cancer, the cells of the F4 group exhibit essentially no response to PHA, while such cells derived from a donor afflicted with cancer respond only to pHA but not to cancer associated antigen or TAA.

Immunologically the SCM responding lymphocytes are T-cell mononuclear leucocytes. Although it is not presently fully understood, it is believed that SCM responding lymphocytes are involved in the recognition of antigens which trigger the body's immune system and are circulating in the blood stream. Accordingly, these cells become primed to recognize foreign substances, such as antigens, produced by the host body's disease or condition and not any antigen in general. Not all lymphocytes found in the blood stream, however, are SCM responding to disease and condition associated substances and only those cells having defined specific buoyant densities are SCM responding. Thus, SCM responding cells having a buoyant density interval at 20°C of $1.0590g/cm^3$ to $1.0670g/cm^3$ (the F2 group) and SCM responding cells having a buoyant density interval at 20°C of $1.0690g/cm^3$ to $1.0730g/cm^3$ (the F4 group) are useful as indicators in the SCM test procedure. Lymphocytes falling outside these densities are not SCM responding in the test procedure described herein and their presence to any appreciable extent should be avoided as diluting the effect of the SCM responding ells which can result in a reduction in test sensitivity.

The term "buoyant density" as used herein is derived from the density of the gradient of the multi-density gradient solution in which a blood component is essentially at zero gravity. That is to say, the component neither exhibits buoyancy nor does it sink in the gradient. Blood components have different buoyant densities thus rendering possible their separation in the multi-density gradient solution.

As already mentioned, the buoyant densities referred to herein are taken at the standard conditions of 20°C and 0.315 to 0.320 Osm/kg. In certain cases it may be inconvenient to operate at standard conditions. In such cases the actual buoyant densities of the blood components which are separated at non-standard conditions are calcualted back to standard conditions according to the following equations:

$$D_X = D_{0.315} + 0.063(x-0.315); \text{ and}$$
$$D_{TO} = D_{20°C} + 2.8 \times 10^{-4} (20°C - T°C); \text{ where}$$

$D_X$ and $D_T$ are buoyant densities at the osmolality of x Osm/kg and T°C, respectively.

F2 and F4 lymphocytes, when maintained in a controlled environment, exhibit an intracellular fluorescein fluorescence polarization peak at 510nm, or 527nm, on excitation at 470nm, or 442nm respectively. In contrast, under the same conditions SCM non-responding lymphocytes do not exhibit an intracellular fluorescein fluorescence polarization peak at 510nm or 527nm.

From the foregoing it should be understood that the separation of SCM responding lymphocytes is a crucial step in the SCM test procedure because the incorporation of SCM non-responders will result in a loss of sensitivity. In addition, poor separation can result in the incorporation of substances with the SCM responding cells which may interfere with their SCM responses. In addition, when testing for cancer the combination of SCM cells from the F2 and the F4 cell groups in the same test sample may produce erroneous results, including false negatives, since, as pointed out above, F2 and F4 SCM responding cells produce different SCM responses and in the case of F4 cells derived from cancer patients, the response to pHA is the same as the response of F2 SCM responding cells from donors not afflicted with cancer.

The separation method of the invention employs a multi-density gradient solution to effect the separation of lymphocytes within narrowly defined density intervals as required by the SCM test procedure outlined above. The density gradients of the fluid must bracket the density intervals of either the F2 SCM responding cells or the F4 SCM responding cells, or both, depending on which cells are to be collected. The multi-gradient density solution is preferably formed by centrifuging a solution of an inert colloidal material such as polyvinyl pyrrolidone coated silica at sufficient speed to form a solution having a density profile which continuously increases between the limiting densities. The multi-gradient density solution may also be formed by programmed mixing of two density limiting fluids of equal osmolalities. Alternatively, a step gradient can be formed by layering fluids with densities corresponding to the buoyant density intervals of F2 and/or the F4 lymphocytes.

In accordance with the invention, the collection and separation of lymphocytes is effected by layering an aliquot of a blood sample on the multi-density gradient solution and centrifuging the combination at sufficient speed to separate the blood components in the density solution according to their respective buoyant densities. The multi-density gradient solution must be inert with respect to the SCM responding lymphocytes.

After centrifugation, consecutive equivolume aliquots of the density fluid are withdrawn by aspiration or titration and the density determined. At a temperature of 20° and an osmolality of 0.315-0.320 osm/kg, selected as and hereinafter referred to as the standard temperature and osmolality, SCM responding cells are contained in the aliquots having densities of between $1.0590g/cm^3$ and $1.0670g/cm^3$ (F2) and in the aliquots having densities of between $1.0690g/cm^3$ and $1.0730g/cm^3$ (F4).

It will be understood that with respect to a continuous density gradient solution, the density profile of the density gradient solution is dependent upon the centrifugation speed, temperature and osmolality of the solution. We have found that the desired continuous density profile can be achieved by centrifuging the solution at $26,000g_{AV}$ using a fixed 29° angle rotor at the standard conditions of temperature and osmolality. With a 34° angle rotor the same density profile is obtained by centrifuging at $11,400g_{AV}$. As previously mentioned, the solution, be it a continuous or a step multi-gradient solution, must have a density gradient profile with a minimum limiting density of not more than $1.050g/cm^3$ which increases to at least $1.070g/cm^3$ to effect the separation of the F2 SCM responding cells and a minimum limiting density of not more than about $1.060g/cm^3$ which continuously increases to about $1.080g/cm^3$ to effect separation of the F4 SCM responding cells. The density profile of the multi-density solution may be sufficiently broad to separate both the F2 and the F4 SCM responding cells in the same density solution.

The separation method of the invention is readily adaptable to automation and the method does not require a higher degree of skill and training beyond the level of one of ordinary skill in the art. The precise control of temperature and fluid osmolality at which the separation is carried out is not required and good results are achieved with temperature control of about ±2°C and osmolality control of about ±0.005 osm/kg. The ability to separate SCM responding cells within narrowly defined buoyant density intervals and the recognition of the more precise buoyant density range of the SCM responding cells substantially eliminates interference by SCM non-responding blood components, thus rendering the SCM test procedure more sensitive, reproducible and less subject to erroneous results than was previously possible using the crude separations obtained prior to the invention. Thus, it is to be understood that, whereas the following examples illustrate a method of carrying out the present invention, these examples are given for purposes of illustration and not of limitation.

### Example 1

#### Preparation of the continuous density gradient fluid

A continuous density gradient solution was prepared by mixing 56 parts by volume of a 9 weight percent solution of PVP coated colloidal silica (Percoll® marketed by Pharmacia AB) in sterile water with 44 parts by volume of a saline solution comprising 0.34 M NaCl (analytical grade) in sterile water. The saline solution also contains $0.2g$ $KH_2PO_4$ per liter of solution. The sterile water and the solutions formed therewith contain no preservatives. The specific density of the solution prior to centrifugation was checked with a Paar Digital Density Meter, Model DMA 55, and was found to average about $1.077g/cm^3$ at 20°C. The solution was sterilized by filtration through a 0.22 micron Millipore filter. The solution was prepared in 1000ml quantities and can be stored for up to 2 months in a dark bottle at 4°C.

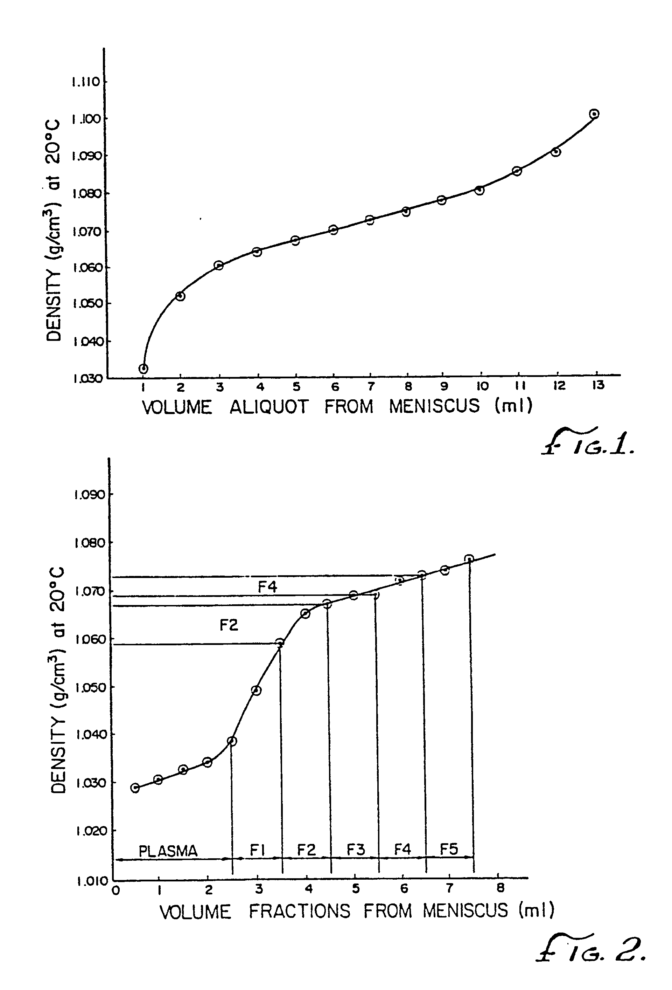

Fifteen milliliter aliquots of the solution were transferred to glass centrifuge tubes (Corex No. 8441, 16mm O.D.) and brought to a temperature of 20°C. The aliquots were centrifuged for 30 minutes at $26,000g_{AV}$ using a fixed 20° angle rotor. The density profile of the solution after centrifugation was determined by determining the absolute density of each consecutive 1ml fraction at 20°C using the Paar Digital Density Meter. A plot of the average density of each of the consecutive segments is shown in Fig. 1. As shown, there is a linear, progressive increase in the density of the fractions beginning with the second volume fraction at a density of about $1.050g/cm^3$ to the twelfth volume fraction at a density of about $1.090g/cm^3$.

### Example 2

#### Separation of SCM responding lymphocytes

Twenty milliliter samples of peripheral blood were obtained from 30 healthy donors and 20 donors who have been positively diagnosed as having a malignancy. For the purposes of the example, the specific type of cancer is not critical.

The blood samples were collected in LH/10 lithium heparine containing blood collection tubes distributed by Searle or heparinized Vacutainer tubes. Heparinized blood samples may be stored at room temperature (18°C to 20°C) for up to 12 hours. Ten milliliters of each blood sample was transferred to a 20ml glass vial containing 0.1g carbonyl-iron powder (type SF distributed by GAF, Great Britain, Ltd.). Equivalent results are achieved using iron powder distributed by Koch-Light Laboratories, Ltd. and identified as 8365 x 99.5%. The vials were rotated at 30 revolutions per minute for 30 minutes at 37°C and then placed on a magnet for 15-30 minutes to effect separation of the phagocytic cells along with the iron powder from the blood sample.

A four-to-five milliliter aliquot of each of the phagocyte depleted blood sample was transferred to a centrifuge tube containing the continuous density gradient solution prepared in Example 1 and from which the top 4ml of the density gradient solution had been removed. Both the density gradient solution and the blood aliquot were equilibrated to a temperature of 20°C before the transfer. The transfer was accomplished by carefully layering the blood on top of the density gradient solution. The tubes were placed in a centrifuge which was thermostatically controlled to maintain a temperature of about 20°C and gradually accelerated over 1 minute to 550g and held at that speed for 30 minutes. No brake was applied during deceleration. Consecutive 0.5ml aliquots were removed from the tubes and the density of each aliquot was determined at 20°C with the Paar Digital Density Meter.

Examination of consecutive 0.5ml aliquots removed from each of the centrifuge tubes after centrifugation showed that the first five aliquots removed which represented the first 2.5ml contained essentially cell-free plasma. The next two aliquots, 2.5 and 3.0 milliliters of the contents of the tubes, had retained herein about 98% of lymphocytes. These two aliquots were combined and designated fraction 1 (F1). The blood components isolated in the 3.5ml and 4.0ml aliquots of the density gradient solution were found to comprise about 99.0% lymphocytes. These two 0.5ml aliquots were combined as fraction 2 (F2). The third fraction (F3) was a combination of the 4.5ml and 5.0ml aliquots of the density gradient solution and contained about 99% lymphocytes. The retained blood components of the fourth fraction (F4) formed by the combination of the aliquots representing 5.5ml and 6.0ml of the density gradient solution consisted of about 80% lymphocytes while the retained blood components of the fifth fraction (F5) formed by the 6.5ml and 7.0ml aliquots of the density gradient solution comprised about 75% lymphocytes. The remaining aliquots contained mainly erythrocytes and were discarded. The other retained blood components in the fractions F1-F5, in addition to lymphocytes, consisted essentially of erythrocytes, although some granulocytes or platelets were also observed.

The density range of each of the fractions was assigned by the densities of the aliquots forming the fraction. Table 1 is a summary of the density ranges and the percent lymphocytes in the retained blood components retained in fractions 1-5 of the density gradient solution.

## Table 1

| Fraction No. | Aliquot | *Density, gm/cm$^3$ | % Lymphocytes |
|---|---|---|---|
| 1 | 2.5-3.5 | 1.0385-1.0590 | 98 |
| 2 | 3.5-4.5 | 1.0590-1.0670 | 100 |
| 3 | 4.5-5.5 | 1.0670-1.0690 | 99 |
| 4 | 5.5-6.5 | 1.0690-1.0730 | 80 |
| 5 | 6.5-7.5 | 1.0730-1.0760 | 75 |

*Taken at 20°C and osmolality of 0.315 - 0.320 osm/kg.

Example 3

Determination of SCM response to cancer associated antigen

Each fraction obtained in Example 2 was checked for the SCM response of the retained lymphocytes in accordance with the SCM cancer screening procedure as described in greater detail in the article by L. Cercek and B. Cercek entitled "Application of the Phenomenon of Changes in the Structuredness of Cytoplasmic Matrix (SCM) in the Diagnosis of Malignant Disorders: A review," Europ. J. Cancer, Vol. 13, pp. 903-915 (1977).

Each fraction was washed twice with 6-7ml of a 0.9% solution of NaCl in sterile water followed by washing in Dulbecco phosphate buffered saline solution (PBS). Between washes the cell suspension was centrifuged at 500g and the supernatent liquid was decanted. After washing, the cell suspension from each fraction was recombined with PBS and the volume adjusted to form a suspension of about $5 \times 10^6$ cells per milliter. The cell suspension of each fraction was divided into three portions: one portion to be used as a control, a second portion to be incubated with PHA and the third portion to be incubated with cancer associated antigen.

In accordance with SCM test procedure, SCM stimulation of the lymphocytes was accomplished by incubating a 0.5ml moiety of each of the lymphocyte portions with 0.05ml of the mitogen (PHA) or with 0.05ml of the cancer associated antigen for at least 30 minutes. The mitogen was reagent grade PHA obtained from Wellcome Ltd. and was reconstituted and then diluted 1:5 to 1:10 with preservative free sterile water. The cancer associated antigen was obtained in accordance with standard techniques by extraction in PBS from a pool of cancer tissues obtained from various types of malignancies. Thus, for each portion of the cell suspension obtained from each fraction, a control moiety, a moiety stimulated by PHA and a moiety stimulated by cancer associated antigen was prepared as described above.

SCM responses are measured with a fluorescence spectrophotometer equipped with polarization accessories to measure verticially and horizontally polarized eimissions. The excitation monochromator is set at a wavelength of 470nm and the emission monochromator at 510nm. The fluorescence spectrophotometer used in this sample was a Perkin-Elmer MPF-4 instrument equipped with a thermostatically controlled cuvette holder and the measurements were carried out at 27°C. A polarized filter for transmitting only vertically polarized light was mounted between the excitation monochromator and the sample. A second polarization filter was fitted with an automatic filter position changer for moving the filter between a first position for transmitting vertically polarized light and a second position for transmitting horizontally polarized light and the assembly was mounted in the light path between the sample and the emission monochromator. The filters were Polacoat Type 105 U.V. sheet polarizers mounted between quartz discs.

Two-tenths of a milliter of the control or stimulated cell suspension moiety was injected into a beaker containing 3ml of a 0.6uM substrate solution of fluoresceindiacetate (FDA) in sterile PBS. The FDA readily permeates the cell membrane and is converted into fluorescein molecules by enzymatic hydrolysis, a substantial portion of which is retained by viable cells. The measurement temperature of 27°C was selected as a compromise between the rate of FDA hydrolysis and the rate of permeation of fluorescein from the cells into the FDA solution. In addition to temperature control, the reproducibility of the fluorescence polarization values obtained is also dependent upon the osmolality and the pH of the FDA substrate solution. The SCM response of cells decreases when the pH of the FDA substrate solution is less than 7.4. Accordinqly, the pH of the cell suspension should be maintained at or slightly above 7.4.

As osmolality increases, the polarization value of the cells also increases and vice versa. Thus, to ensure reproducibility between samples, the osmolality of the FDA substrate solution should be held to within ±1% of the isotonic value of 0.330 osm/kg.

After injection of the cell suspension into the FDA substrate solution, the combination was immediately transferred to a quartz cuvette and placed in the thermostatically controlled cuvette holder. The intensity of the fluorescence parallel and perpendicular to the vertically polarized exciting light was measured by alternating the orientation of the polarization filter located betwwen the sample and the emission monochromator and the measurements were recorded for about 6 minutes or until the intensity of the fluorescence perpendicular to the plane of the polarized exciting light reach about 80-90% of full scale deflection, whichever first occured. Fluorescein leakage from the cells and background flurorescence is corrected for by filtering the cell suspension under controlled suction (less than 40 cm of Hg) and recording the emissions of the filtrate in the same manner as for the cell suspension. The fluorescence intensities (I) emitted by the cells, are obtained by subtracting the corresponding intensities of the filtrate from the total fluorescence intensities, extrapolated to the half-time of the filtration interval. The polarization or P value of the cells in the sample is calculated from the relatinship:

$$P = I_{||} - GI_{\perp} / I_{||} + GI_{\perp} \; ; \; where$$

$$I_{||} \quad and \quad I_{\perp}$$

are polarized fluorescence intensities parallel and perpendicular respectively to the vertically polarized light source emitted by the cells of the sample and G is a correction factor for the unequal transmission of the parallel and perpendicular components of the polarized light through the optical system.

The value of G is determined by dividing the perpendicular fluorescence intensity by the parallel fluorescence intensity emitted from a filtrate solution or from a $10^{-7}$M solution of fluorescein in PBS which has been excited with horizontally polarized light at 470nm. For the equipment used in this example the value of G was 0.42.

The mean P value for samples stimulated with PHA and cancer associated antigen from each of five fractions of blood obtained from 30 healthy donors and from 20 donors who have been positively diagnosed as having a malignant disorder were reported as a percent of the mean P value of unstimulated control samples from the five fractions of the blood samples. The results as summarized in Table 2 below.

## Table 2

| Fraction No. | SCM Response to PHA & Cancer Associated Antigen as % of Control | | | |
|---|---|---|---|---|
| | *PHA | *Antigen | **PHA | **Antigen |
| 1 | 99.9±1.5 | 100±2.0 | 100±2.0 | 99.8±1.5 |
| 2 | 71 ±6.0 | 99±3.0 | 98±2.0 | 74 ±6.0 |
| 3 | 101 ±2.0 | 100±2.0 | 100±2.0 | 100 ±2.5 |
| 4 | 99 ±2.5 | 100±2.0 | 74±8.0 | 99 ±3.0 |
| 5 | 100 ±1.5 | ------- | 99±2.0 | --------- |

PHA is phytohaemagglutinin;

*     Each value is the mean of 30 samples from healthy donors

**     Each value is the mean of 20 samples from donors diagnosed as having a malignant disorder

It will be apparent from Table 2 that the lymphocytes retained in fractions 1, 3 and 5 of the continuous density gradient fluid are SCM non-responding. There is essentially no response by the cells of fractions 1 and 3 to stimulation by either the mitogen or the antigen and in the case of the cells of fraction 5, the lymphocytes from healthy donors exhibited no response to the mitogen, indicating that these cells are also SCM non-responding. This is confirmed by the fact that the spectrum of these cells does not exhibit a fluorescence polarization peak at 510nm, indicating that these are SCM non-responders as contrasted to the spectrum of F2 and F4 cells, which exhibit a peak at 510nm. The cells retained in fraction 2 (F2 cells) exhibited classic responses to stimulation by the mitogen and the antigen. Thus, F2 lymphocytes of healthy donors stimulated by the mitogen PHA have a marked percentage decrease in the polarization value as compared to the unstimulated control samples. This indicates an SCM response by the lymphocytes to PHA. These same lymphocytes had essentially no SCM response to the cancer associated antigen. By the same token, F2 lymphocytes from donors afflicted with cancer had no SCM response to PHA but a definite response to the cancer associated antigen. This establishes that the F2 lymphocytes, having a buoyant density of 1.0590-1.0670gm/cm³ at 20°C are suitable for use in the SCM tesi for the detection of cancer.

The F4 lymphocytes, that is, the cells having a buoyant density of 1.0690-1.0730g/cm³, also demonstrate an SCM response to PHA which would indicate usefulness in the SCM cancer detection procedure but the response is different than for F2 lymphocytes. In contrast to F2 responders, F4 lymphocytes from donors afflicted with cancer have a significant SCM response to PHA. In addition, F4 cells exhibit no response to cancer associates antigen regardless of the donor's condition. It should be noted that the response of the F4 lymphocytes to PHA is the opposite to the response of F2 cells to the mitogen. While the reason for this is not understood, it will be apparent that the F4 lymphocytes can be used in the SCM test to screen blood samples for the presence of cancer. A positive SCM response of a donor's F4 lymphocytes to PHA will provide a positive indication of the presence of a malignancy, calling for further testing and diagnosis.

It will also be apparent that F4 lymphocytes should be separated from F2 lymphocytes prior to mitogen stimulation since the presence of both F2 and F4 cells may lead to erroneous results, indicating the absence of malignancy when, in fact, a malignancy is present or vice versa. In accordance with the improved separation technique of the invention, F2 and F4 cells are separated and biophysically defined so that the danger of false results due to the presence of either SCM non-responding cells or a mixture of F2 and F4 SCM responders can be prevented.

### Example 4

#### SCM response to determine allograft rejection and tissue compatability

F2 lymphocytes separated in accordance with the procedure of Example 2 from the peripheral blood of an organ transplant recipient are used to indicate the onset of allograft rejection crisis. Utilizing the SCM response technique of Example 3, the separated F2 lymphocytes are incubated with a tissue extract in PBS obtained from tissue of the organ donor. Organ rejection is indicated when the F2 lymphocytes from the organ recipient show a decrease in intracellular fluorescein fluorescence polarization after contact with the tissue extract from the organ donor. Compatibility is indicated by no decrease in the polarization value of the recipient's F2 lymphocytes in response to the donor's tissue extract.

As an alternative to the tissue extract, the F2 lymphocytes of the recipient are incubated with a cell free extract or essentially cell free extract (about 5% cells) in PBS of F2 lymphocytes from the blood of the organ donor and SCM response is measured as in Example 3.

The technique of this Example is also utilized prior to the organ transplant to determine compatibility between the organ donor and the potential organ recipient. The procedure of this Example is carried out in a relatively short time, 3 to 4 hours,, and is utilized as a timesaving, alternative test procedure to the conventional mixed lymphocyte reaction procedure, which is conventionally used as a measure of compatibility between a donor and a recipient and which takes between 48 and 72 hours before results are available.

### Example 5

#### SCM response to determine allergenic reaction

F2 lymphocytes are separated in accordance with Example 2 from the peripheral blood of a donor suffering from an allergenic reaction and are used in accordance with the method of Example 3 to determine the reaction of the donor to allergens such as pollen extract, dust extract, animal hair extract, grass extracts, bee toxins and the like as are presently used in conventional skin patch tests. Allergenic reaction to an allergen is indicated by the method of Example 3 by a decrease in the intracellular fluorescein fluorescence polarization value of the donor's F2 lymphocytes which indicates an SCM response by the F2 lymphocytes to a particular allergen. In accordance with the procedure of this Example, the painful and otherwise inconvenient skin patch tests can be avoided.

### Example 6

#### SCM response to determine viral and bacterial infection

F2 lymphocytes are isolated in accordance with the method of Example 2 from the peripheral blood of a donor afflicted with an unknown viral or bacterial infection. The F2 cells are incubated in accordance with the method of Example 3 with extracts and toxins of virus or bacteria which are suspected to be the cause of the infection. The F2 lymphocytes will respond as indicated by a decrease in intracellular fluorescein fluorescence polarization when incubated with the specific toxin which is present in the body of the donor and not to any other agents with which the F2 lymphocytes are incubated.

## Claims

1. A procedure for testing a sample of blood for the presence of a condition or disease in a donor of the blood based upon the response of lymphocytes separated from a blood sample to contact with a substance associated with the condition being tested for, the lymphocyte response being indicated by changes in the structuredness of cytoplasmic matrix of said lymphocytes as a result of contact between said lymphocytes and said substance, **characterised in that said** lymphocytes have buoyant densities of 1.0590 g/cm$^3$ to 1.0670 g/cm$^3$ at 20°C in a multi-density gradient solution having an osmolality of 0.315 Osm/kg .

2. A procedure according to claim 1 wherein said condition or disease associated substance is a cancer associated antigen.

3. A procedure according to Claim 1 wherein said condition or disease associated substance is selected from viral associated antigens and bacterial associated antigens.

4. A procedure according to claim 1 wherein said condition or disease associated substance is an antigen capable of causing a decrease in the structuredness of the cytoplasmic matrix of lymphocytes incompatible with the donor of the lymphocytes producing the substance and present as an indicator of allograft rejection crisis.

5. A procedure according to claim 1 wherein said condition or disease associated substance is tissue extract from a transplant donor whereby compatibility for organ transplantation between a transplant recipient from which the blood sample is obtained, and the transplant donor from which the tissue extract is obtained, is determined.

6. A method for classifying and separating from a blood sample lymphocytes capable of responding to stimulation by a condition or disease associated substance, said response being exhibited by measurable changes in the structuredness of the cytoplasmic matrix of said lymphocytes responsive to contact between said lymphocytes and said substance, said method comprising the steps of:

a) forming a multi-density gradient solution having a minimum density of no greater than 1.050 g/cm³ and a maximum density of no less than 1.080 g/cm³ at 20°C;

b) introducing a sample of blood to said multi-density gradient solution;

c) centrifuging said blood and said multi-density gradient solution at a controlled temperature and at sufficient effective gravitational force to cause the components of said blood sample to separate and be distributed and retained in said multi-density gradient solution according to the buoyant densities of said blood components;

d) separating aliquots of said multi-density gradient solution, including the retained blood components in each said aliquot;

e) collecting an aliquot of the multi-density gradient solution in discrete fractions, the aliquot containing the portion of the multi-density gradient solution having densities of from 1.0590 g/cm³ to 1.0670 g/cm³; and

f) subjecting said aliquot to a washing operation, thereby to obtain a suspension of lymphocytes having buoyant densities of between 1.0590 g/cm³ and 1.0670 g/cm³.

7. A method according to claim 6 wherein said multi-density gradient solution comprises a continuous density gradient.

8. A method according to claim 6 wherein said multi-density gradient solution has an osmolality of 0.315 to 0.320 Osm/kg.

9. A method according to claim 6 wherein said blood and said multi-density gradient solution are centrifuged at an effective gravitational force of 550 g at a temperature of 20°C.

10. A method according to claim 6 wherein said multi-density gradient solution comprises an aqueous solution of colloidal silica diluted with saline solution.

11. A method according to claim 10 wherein said colloidal silica is coated with an inert material.

12. A method according to claim 10 wherein said aqueous solution of colloidal silica diluted with saline solution is centrifuged at a temperature of 20°C and at a effective gravitational force of about 26,000 g using a fixed 29° rotor.

13. A method according to claim 10 wherein said aqueous solution of colloidal silica diluted with saline solution is centrifuged at a temperature of 20°C and at a effective gravitational force of about 11,400 g using a fixed 34° rotor.

14. A method according to claim 6 wherein said multi-density gradient solution comprises a mixture of at least two fluids of equal osmolality, the maximum density of the gradient being the density of the most dense fluid and the minimum density of the gradient being the density of the least dense fluid.

15. A method according to claim 6 further comprising the steps of:

g) collecting a second aliquot of the multi-density gradient solution in discrete fractions, the second aliquot containing the portion of the multi-density gradient solution having densities of from 1.0690 g/cm³ to 1.0730 g/cm³; and

h) subjecting said second aliquot to a washing operation, thereby to obtain a second suspension of lymphocytes having buoyant densities between 1.0690 g/cm³ and 1.0730 g/cm³.

16. A procedure for testing a sample of blood for the presence of cancer in a donor of the blood based upon the responsive of lymphocytes separated from a blood sample to contact with a substance associated with cancer, the lymphocyte response being indicated by changes in the structuredness of cytoplasmic matrix of said lymphocytes as the result of contact between said lymphocytes and said substance, **characterised in that** said lymphocytes have buoyant densities of 1.0690 g/cm³ to 1.0730 g/cm³ at 20°C in a multi-density gradient solution having an osmolality of 0.315 Osm/kg.

## Patentansprüche

1. Verfahren zum Testen einer Blutprobe auf das Vorliegen eines Zustandes oder einer krankheit in einem Donor (Spender) des Blutes, auf der Grundlage des Ansprechverhaltens von aus einer Blutprobe abgetrennten Lymphocyten auf Kontakt mit einer dem zu testenden Zustand zugeordneten Substanz, wobei das Ansprechverhalten der Lymphocyten durch Änderungen in der Strükturiertheit der Cytoplasma-Matrix der genannten Lymphocyten als Ergebnis des Kontakts zwischen den gennanten Lymphocyten and der gennanten Substanz angezeigt wird, dadurch **gekennzelchnet** , daß die genannten Lymphocyten Auftriebsdichten von 1,0590 g/cm³ bis 1,0670 g/cm³ bei 20°C in einer Multi-Dichte-Gradienten-Lösung mit einer Osmolalität von 0,315 Osm/kg aufweisen.

2. Verfahren nach Anspruch 1, bei welchem die genannte dem Zustand oder der krankheit zugeordnete Substanz ein mit krebs verbundenes Antigen ist.

3. Verfahren nach Anspruch 1, bei welchem die genannte, dem Zustand oder der krankheit zugeordnete Substanz aus Viren zugeordneten Antigenen und Bakterien zugeordneten Antigenen gewählt ist.

4. Verfahren nach Anspruch 1, bei welchem die genannte, dem Zustand oder der Erkrankung zugeordnete Substanz ein Antigen ist, das eine Verringerung in der Strukturiertheit der Cytoplasmamatrix von Lymphocyten hervorzurufen vermag, die inkompatibel mit dem Donor der die Substanz erzeugenden Lymphocyten ist und als ein Indikator für eine Allo-Pfropf-Abweisungs-krise vorliegt.

5. Verfahren nach Anspruch 1, bei welchem die genannte, dem Zustand oder der krankheit zugeordnete Substanz ein Gewebeextrakt von einem Transplantationsdonor ist, derart daß die kompatibilität für eine Organverpflanzung zwischen einem Transplantations-Empfänger, von welchem die Blutprobe abgenommen wird, und dem Transplantationsdonor, von welchem der Gewebeextrakt erhalten wurde, bestimmt wird.

6. Verfahren zum klassifizieren und Abtrennen von Lymphocyten, die auf Stimulation durch eine einem Zustand oder einer krankheit zugeordnete Substanz anzusprechen vermögen, aus einer Blutprobe, wobei das genannte Ansprechverhalten sich durch meßbare Änderungen in der Strukturiertheit der Cytoplasmamatrix der genannten Lymphocyten in Abhängigkeit von einem kontakt zwischen den genannten Lymphocyten und der genannten Substanz äußert, wobei das genannte Verfahren die Schritte umfaßt:

(a) Bilden einer Multi-Dichte-Gradienten-Lösung mit einer Mindestdichte von nicht mehr als 1,050 g/cm³ und einer Maximaldichte von nicht weniger als 1,080 g/cm³ bei 20°C;

(b) Einführen einer Blutprobe in die genannte Multi-Dichte-Gradienten-Lösung;

(c) Zentrifugieren des genannten Bluts und der genannten Multi-Dichte-Gradienten-Lösung bei einer kontrollierten Temperatur bei einer ausreichenden wirksamen Gravitationskraft, um eine Trennung, Verteilung und Zurückhaltung der komponenten der genannten Blutprobe in der genannten Multi-Dichte-Gradienten-Lösung nach Maßgabe der Auftriebsdichten der genannten Blutkomponenten zu bewirken;

(d) Abtrennen von Teilmengen der genannten Multi-Dichte-Gradienten-Lösung, einschließlich der jeweils in den einzelnen Teilmengen enthaltenen Blutkomponenten;

(e) Aufsammeln einer Teilmenge der Multi-Dichte-Gradienten-Lösung in diskreten Fraktionen, wobei die Teilmenge den Teil der Multi-Dichte-Gradienten-Lösung mit Dichten von 1,0590 g/cm³ bis 1,0670 g/cm³ enthält; sowie

(f) die genannte Teilmenge wird einem Waschvorgang unterzogen, wodurch eine Suspension von Lymphocyten mit Auftriebsdichten zwischen 1,0590 g/cm³ und 1,0670 g/cm³ erhalten wird.

7. Verfahren nach Anspruch 6, bei welchem die genannte Multi-Dichte-Gradienten-Lösung einen kontinuierlichen Dichtegradienten umfaßt.

8. Verfahren nach Anspruch 6, bei welchem die genannten Multi-Dichte-Gradienten-Lösung eine Osmolalität von 0,315 bis 0,320 Osm/kg besitzt.

9. Verfahren nach Anspruch 6, wobei das genannte Blut und die genannte Multi-Dichte-Gradienten-Lösung bei einei wirksamen Gravitationskraft von 550 g bei einer Temperatur von 20°C zentrifugiert werden.

10. Verfahren nach Anspruch 6, bei welchem die genannte Multi-Dichte-Gradienten-Lösung eine wäßrige Lösung von kolloidalem Siliziumoxid verdünnt mit Salzlösung umfaßt.

11. Verfahren nach Anspruch 10, bei welchem das genannte kolloidale Siliziumoxid mit einem inerten Material überzogen ist.

12. Verfahren nach Anspruch 10, bei welchem die genannte mit Salzlösung verdünnte Lösung von kolloidalem Siliziumoxid bei einer Temperatur von 20°C und unter einer wirksamen Gravitationskraft von etwa 26.000 g unter Verwendung eines 29° Festwinkelrotors zentrifugiert wird.

13. Verfahren nach Anspruch 10, bei welchem die genannte mit Salzlösung verdünnte Lösung von kolloidalem Siliziumoxid bei einer Temperatur von 20°C und unter einer wirksamen Gravitationskraft von etwa 11.400 g unter Verwendung eines 34° Festwinkelrotors zentrifugiert wird.

14. Verfahren nach Anspruch 6, bei welchem die genannte Multi-Dichte-Gradienten-Lösung ein Gemisch von wenigstens 2 Strömungsmitteln gleicher Osmolalität umfaßt, wobei die maximale Dichte des Gradienten die Dichte des dichtesten Strömungsmittels und die minimale Dichte des Gradienten die Dichte des Strömungsmittels mit der geringsten Dichte sind.

15. Verfahren nach Anspruch 6, des weiteren umfassend die Verfahrensschritte:

(g) Aufsammeln einer zweiten Teilmenge der Multi-Dichte-Gradienten-Lösung in diskreten Fraktionen, wobei die zweite Teilmenge den Bereich der Multi-Dichte-Gradienten-Lösung mit Dichten von 1,0690 g/cm³ bis 1,0730 g/cm³ enthält; und

(h) die genannte zweite Teilmenge wird einem Waschvorgang unterzogen, wodurch eine zweite Suspension von Lymphocyten mit Auftriebsdichten zwischen 1,0690 g/cm³ und 1,0730 g/cm³ erhalten wird.

16. Verfahren zum Testen einer Blutprobe auf das Vorliegen von krebs in einem Donor des Blutes, auf der Grundlage des Ansprechverhaltens von aus einer Blutprobe abgetrennten Lymphocyten auf kontakt mit einer dem krebs zugeordneten Substanz, wobei das Ansprechverhalten der Lymphocyten durch Änderungen in der Strukturiertheit der Cytoplasmamatrix der genannten Lymphocyten als Ergebnis des kontakts zwischen den genannten Lymphocyten und der genannten Substanz angezeigt wird, dadurch **gekennzeichnet**, daß die genannten Lymphocyten Auftriebsdichten von 1,0690 g/cm³ bis 1,0730 g/cm³ bei 20°C in einer Multi-Dichte-Gradienten-Lösung mit einer Osmolalität von 0,315 Osm/kg aufweisen.

## Revendications

Procédé pour contrôler un échantillon de sang pour la présence d'une pathologie ou d'une maladie chez un donneur de sang, basé sur la réponse des lymphocytes séparés d'un échantillon de sang en contact avec une substance associée à la pathologie pour laquelle elle est testée, la réponse lymphocytaire étant indiquée par des modifications dans la structure de la matrice cytoplasmique des lymphocytes précités à la suite du contact entre les lymphocytes précités et la substance précitée, caractérisé en ce que les lymphocytes précités ont des densités flottables de 1,0590 g/cm³ à 1,0670 g/cm³ à 20°C dans une solution de gradient multidensité ayant une osmolalité de 0,315 Osm/kg.

2. Procédé selon la revendication 1, où la substance précitée associée à une pathologie ou à une maladie est un antigène associé à un cancer.

3. Procédé selon la revendication 1, où la substance précitée associée à une pathologie ou à une maladie est sélectionnée parmi des antigènes associés à un virus et des antigènes associés à une bactérie.

4. Procédé selon la revendication 1, où la substance précitée associée à une pathologie ou à une maladie est un antigène capable d'entraîner une diminution dans la structure de la matrice cytoplasmique des lymphocytes incompatible avec le donneur de lymphocytes produisant la substance et présent comme indicateur d'une crise du rejet d'allogreffe.

5. Procédé selon la revendication 1, où la substance précitée associée à une pathologie ou à une maladie est un extrait de tissu d'un donneur de greffe au moyen duquel la compatibilité pour une transplantation d'organe entre un receveur de greffe à partir duquel l'échantillon de sang est obtenu, et le donneur de greffe à partir duquel l'extrait de tissu est obtenu, est déterminée.

6. Procédé pour classer et séparer à partir d'un échantillon de sang des lymphocytes capables de répondre à une stimulation par une substance associée à une pathologie ou à une maladie, la réponse précitée étant montrée par des modifications mesurables dans la structure de la matrice cytoplasmique des lymphocytes précités sensible au contact entre les lymphocytes précités et la substance précitée, le procédé précité comprenant les étapes :

a) de formation d'une solution de gradient de multi-densité ayant une densité minimale de pas plus de 1,050 g/cm³ et une densité maximale de pas moins de 1,080 g/cm³ à 20°C ;

b) d'introduction d'un échantillon de sang dans la solution de gradient de multidensité ;

c) de centrifugation du sang précité et de la solution du gradient de multidensité à une température contrôlée et à une force de gravitaiion efficace suffisante pour que les composants de l'échantillon de sang précité soient séparés et soient distribués et retenus dans la solution de gradient de multidensité précitée selon les densités flottables des composants prédits du sang;

d) de séparation des aliquots de la solution de gradient de multidensité précitée,incluant les composants du sang retenus dans chaque aliquot précité ;

e) de récupération d'un aliquot de la solution de gradient de multidensité en fractions discrètes, l'aliquot contenant la partie de la solution de gradient de multidensité ayant des densités de 1,0590 g/cm³ à 1,0670 g/cm³ ; et

f) de soumission de l'aliquot précité à une opération de lavage, pour obtenir ainsi une suspension de lymphocytes ayant des densités flottables entre 1,0590/cm³ et 1,0670 g/cm³.

7. Procédé selon la revendication 6, où la solution précitée de gradient de multidensité comprend un gradient de densité continu.

8. Procédé selon la revendication 6, où la solution précitée de gradient de multidensité a une osmolalité de 0,315 à 0,320 Osm/kg.

9. Procédé selon la revendication 6, où le sang précité et la solution de gradient de multidensité précitée sont centrifugés à une force de gravitation efficace de 550 g à une température de 20°C.

10. Procédé selon la revendication 6, où la solution de gradient de multidensité précitée comprend une solution aqueuse de silice colloïdale diluée avec une solution saline

11. Procédé selon la revendication 10, où la silice colloïdale précitée est recouverte d'un matériau inerte.

12. Procédé selon la revendication 10, où la solution aqueuse précitée de silice colloïdale diluée à une solution saline est centrifugée à une température de 20°C et à une force de gravitation efficace d'environ 26.000 g en utilisant un rotor fixe de 29°.

13. Procédé selon la revendication 10, où la solution aqueuse précitée de silice colloïdale diluée avec une solution saline est centrifugée à une température de 20°C et à une force de gravitation efficace d'environ 11.400 g en utilisant un rotor fixe de 34°.

14. Procédé selon la revendication 6, où la solution précitée de gradient de multidensité comprend un mélange-d'au moins deux fluides d'osmolalité égale, la densité maximale du gradient étant la densité du fluide le plus dense et la densité minimale du gradient étant la densité du fluide le moins dense.

15. Procédé selon la revendication 6, comprenant de plus les étapes :

g) de récupération d'un second aliquot de la solution de gradient de multidensité en iractions discrètes, le second aliquot contenant la partie de la solution de gradient multidensité ayant des densités de $1,0690/cm^3$ à $1,0730$ g/cm$^3$;

h) de soumission du second aliquot à une opération de lavage, pour obtenir ainsi une seconde suspension de lymphocytes ayant des densités flottables entre $1,0690$ g/cm$^3$ et $1,0730$ g/cm$^3$.

16. Procédé pour contrôler un échantillon de sang pour la présence de cancer chez un donneur de sang basé sur les lymphocytes séparés d'un échantillon de sang sensibles au contact avec une substance associée au cancer, la réponse du lymphocyte étant indiquée par des modifications de la structure de la matrice cytoplasmique des lymphocytes précités, résultat du contact entre les lymphocytes précités et la substance précitée, caractérisé en ce que les lymphocytes précités ont des densités flottables de $1,0690$ g/cm$^3$ à $1,0730$ g/cm$^3$ à 20°C dans une solution de gradient de multidensité ayant une osmolalité de 0,315 Osm/kg.

FIG.1.

FIG. 2.